# EUROPÄISCHE PATENTSCHRIFT

(11) **EP 0 670 145 B1**
(45) Veröffentlichungstag und Bekanntmachung des Hinweises auf die Patenterteilung: **08.03.2000**
(21) Anmeldenummer: 94117453.4
(22) Anmeldetag: 04.11.1994
(51) Int. Cl.: A61B 6/00, H05G 1/02

(54) **Röntgenuntersuchungsausrüstung**
Equipment for X-ray examination
Equipement pour la radiographie

(30) Priorität: 22.12.1993 SE 9304249
(43) Veröffentlichungstag der Anmeldung: 06.09.1995
(73) Patentinhaber: Siemens-Elema AB, 171 95 Solna 1 (SE)
(72) Erfinder: Stenfors, Per, S-163 58 Spanga (SE)

(56) Entgegenhaltungen:
- EP-A- 0 224 886
- EP-A- 0 375 552
- US-A- 4 922 512

## Beschreibung

Die Erfindung bezieht sich auf eine Röntgenuntersuchungsausrüstung mit einem Stativ, bestehend aus mindestens einem Fundament, einer um eine horizontale Achse drehbaren und mit dem Fundament verbundenen Halterung für einen gebogenen Träger, an dem eine Röntgenröhre und ein Rezeptor befestigt sind, einer Befestigungsvorrichtung für das Stativ und aus einem Arm, der das Stativ mit der Befestigungsvorrichtung verbindet, wobei das eine Ende des Armes um eine Achse an der Befestigungsvorrichtung drehbar ist und das andere Ende des Armes mit einer am Fundament angeordneten vertikalen Achse drehbar verbunden ist sowie mit einem Untersuchungstisch, der zumindest entlang einer Zentrumlinie verschiebbar ist.

In Verbindung mit Herzuntersuchungen ist es von Bedeutung, dass die Röntgenröhre und der Rezeptor den Oberkörper des Patienten bis zu den Leisten überstreichen können. Es ist ausserdem bei anderen Untersuchungen wünschenswert, die gesamte Länge des Patienten zu überstreichen. Bei solchen Untersuchungen kann das Stativ an der Längsseite des Untersuchungstisches angebracht sein und mit dem Untersuchungstisch parallel verschoben werden, was bedeutet, dass der erwähnte Patientenbereich mit der Röntgenröhre und dem Rezeptor ohne weiteres erreicht wird. Eine solche Untersuchungsausrüstung ist durch den Siemens-Prospekt "ANGIOSTAR" bekannt, bei dem das Stativ an Bodenschienen verschiebbar ist. Der Arzt hat bei solchen Untersuchungsausrüstungen am Kopfende und an der einen Längsseite des Tisches einen sehr guten Zugang zum Patienten, aber selbstverständlich nicht von der Seite, an der das Stativ angebracht ist.

Zu einer anderen Art von Röntgenuntersuchungsausrüstungen zählt ein am Kopfende angebrachtes Stativ, bei dem der Innendurchmesser des gebogenen Trägers bestimmt, wieweit die Röntgenröhre und der Rezeptor den Körper des Patienten überstreichen können, wenn das Kopfende des Untersuchungstisches in den Trägerbogen zwischen der Röntgenröhre und dem Rezeptor dicht an der Halterung des Trägerbogens plaziert ist. Eine solche Untersuchungsausrüstung ist in dem Siemens-Prospekt "COROSKOP HS" gezeigt und beschrieben. Bei dieser Untersuchungsausrüstung ist das Problem mit der durch die Form des Trägerbogens erwähnten Bewegungsbeschränkungen der Röntgenröhre und des Rezeptors teilweise gelöst, indem das Stativ, das am Boden plaziert ist, um eine vertikale Achse drehbar ist und mit einem an der Decke aufgehängten Untersuchungstisch, der in seiner Längsrichtung und seitlich zur Längsrichtung verschoben werden kann oder mit einem an Boden angebrachten Untersuchungstisch, der um eine vertikale Achse drehbar ist, kombiniert werden kann. Bei jeder Drehung des Statives um die vertikale Achse muss die Lage des Untersuchungstisches eingestellt werden.

In dem Prospekt "Advantx L/C" der Fa GE Medical Systems ist eine Röntgenuntersuchungsausrüstung beschrieben. Die Achse der Befestigungsvorrichtung, um die der Arm drehbar verbunden ist, ist am Boden appliziert und von oben betrachtet auf der fiktiven Zentrumlinie des Untersuchungstisches unterhalb des Kopfendes des Tisches angebracht. Durch den Arm, der sich unter den Tisch erstreckt, kann das Stativ mit dem Träger für die Röntgenröhre und den Rezeptor um die Achse der Befestigungsvorrichtung von einer Lage, in der der Träger am Kopfende des Tisches angebracht ist, in einer weiteren Lage, in der der Träger mit einem Winkel von 90° im Verhältnis zur Längsrichtung des Tisches angeordnet ist, gedreht werden. Das Stativ ist derart aufgebaut, dass die Zentrumlinie zwischen der Röntgenröhre und dem Rezeptor bei einer vertikalen Einstellung mit der Achse der Befestigungsvorrichtung achsfluchtend liegt. Daher liegt die fiktive Zentrumlinie zwischen der Röntgenröhre und dem Rezeptor bei einer Drehung des Statives um die Achse der Befestigungsvorrichtung im gleichen Isozentrum immer fest. Die Grösse der Längsverschiebung des Tisches hängt davon ab, wie gross der Teil des Patienten ist, der untersucht werden kann. Da der am Boden plazierte Arm im Bereich der Achse der Befestigungsvorrichtung verhältnismässig breit und hoch ist, kann der Arzt während einer Untersuchung mit den Füssen an den Arm stossen, was störend sein kann. Durch die Lage und Stärke des Armes kann der Träger für die Röntgenröhre und den Rezeptor bei einer Vertikallage nicht bis zum Boden herabgesenkt werden. Bei einer solchen Vertikallage können in einigen Fällen eine Herabsenkung der Röntgenröhre und des Rezeptors mit 4 bis 5 cm, was schätzungsweise die Stärke des Armes ist, entscheidend sein, um eine gute Aufnahme zu erhalten.

In der US-A-4 922 512 ist eine Röntgenuntersuchungsausrüstung der eingangs genannten Art beschrieben. Die Achse der Befestigungsvorrichtung, um die der Arm drehbar verbunden ist, ist genau wie die im Prospekt "Advantx L/C" der Fa GE Medical Systems beschriebene Röntgenausrüstung am Boden appliziert und von oben betrachtet auf der fiktiven Zentrumlinie des Untersuchungstisches unterhalb des Kopfendes des Tisches angebracht und weist daher die Nachteile, die in Verbindung mit dem Prospekt "Advantx L/C" bereits ausführlich beschrieben worden sind, auf. Durch die Plazierung der Befestigungsvorrichtung des Armes unterhalb des Tisches muß der Arm außerdem verhältnismäßig lang gehalten werden, damit der Zentralstrahl der Röntgenröhre und des Rezeptors die Zentrumlinie des Untersuchungstisches schneiden kann. Beim Schwenken des Armes um die Befestigungsvorrichtung, z.B. von einer Kopflage in einer Seitenlage des Statives, wird durch die Länge des Armes sehr viel Platz benötigt. Die Art wie die Bewegungen des Stativs gesteuert werden, ist hier nicht beschrieben.

In dem Philips-Prospekt "Integris BN 3000 und Integris BV 3000" ist ein weiteres Stativ dargestellt und beschrieben, das zwischen einer Kopf- und einer Seitenlage wechseln kann. Dieses Stativ ist derart aufgebaut, dass die Halterung für den Träger, an dem die Röntgenröhre und der Rezeptor befestigt sind, direkt an der Befestigungsvorrichtung am Boden angebracht und um deren vertikalen Achse drehbar ist. Die Achse der Befestigungsvorrichtung ist unter dem Kopfende des Untersuchungstisches angeordnet und, von oben betrachtet, an der fiktiven Zentrumlinie des Untersuchungstisches plaziert. Die Zentrumlinie der Röntgenröhre und des Rezeptors ist bei einer Vertikallage auch hier mit der Achse der Befestigungsvorrichtung achsfluchtend angeordnet. Durch diesen Aufbau verlässt die Zentrumlinie für die Röntgenröhre und den Rezeptor bei einer Schwenkung der Halterung und des Trägers nicht ein fiktives Isozentrum. Auch hier hängt es von der Grösse der Längsverschiebung des Tisches ab, wie gross der Teil des Patienten ist, der untersucht werden kann. Durch den Aufbau des Statives kann dieser Träger auch nicht bis zum Boden herabgesenkt werden.

Der Erfindung liegt die Aufgabe zugrunde, eine Röntgenuntersuchungsausrüstung der eingangs genannten Art mit einem im Aufbau verhältnismässig einfachen Stativ zu schaffen, das eine ausserordentlich grosse Reichweite aufweist und das ohne Hilfe einer Tischverschiebung die Zentrumlinie der Röntgenröhre und des Rezeptors zwischen zwei verhältnismäßig weit außeinanderliegenden Punkten auf der Zentrumlinie des Untersuchungstisches versetzen kann.

Diese Aufgabe ist erfindungsgemäss dadurch gelöst, dass die Befestigungsvorrichtung so plaziert ist, dass deren Achse ausserhalb der Zentrumlinie des Untersuchungstisches liegt und daß Mittel vorgesehen sind, die bei einer Drehung des Armes um die Achse der Befestigungsvorrichtung das Stativ um die Achse am Fundament derart drehen, dass die Zentrumlinie der Röntgenröhre und des Rezeptors die Zentrumlinie des Untersuchungstisches in mindestens zwei Armwinkellagen schneidet, wobei in der ersten Armwinkellage das Stativ, von oben betrachtet, mit der Zentrumlinie des Untersuchungstisches achsfluchtend liegt und in der zweiten Armwinkellage das Stativ mit dieser Zentrumlinie einen Winkel bildet. Hierdurch kann das Stativ ein übliches in seinem Aufbau sehr einfaches und billiges kopfstellbares Stativ, z.B. ein C-Bogentyp, sein. Das Stativ nimmt verhältnismässig wenig Platz ein und stört den Arzt nicht, da das Fundament ausserhalb des eigentlichen Bereiches um den Untersuchungstisch herum angebracht ist, wodurch der Arzt einen sehr guten Zugang zum Patienten erhält.

In einer vorteilhaften Weiterbildung der Erfindung wird vorgeschlagen, dass die Drehung des Statives um den Arm im Vergleich zur Drehung des Armes um die Befestigungsvorrichtung mit einer derartigen Übersetzung ausgeführt wird, dass der Schnittpunkt zwischen der Zentrumlinie der Röntgenröhre und des Rezeptors und der Zentrumlinie des Untersuchungstisches in der ersten Armwinkellage gegenüber dem Schnittpunkt in der zweiten Armwinkellage verschoben ist. Durch diese Übersetzung wird erreicht, dass das Stativ eine sehr grosse Längshub bis zu etwa 45 cm erhält. Einschliesslich eines Rezeptorhubs von 17 cm braucht daher der Längshub des Untersuchungstisches lediglich etwa 125 cm zu sein, um eine Ganzkörperuntersuchung eines Patienten mit einer Länge von 187 cm durchführen zu können.

Nach einer Weiterbildung der Erfindung ist die erwähnte Übersetzung zwischen der Drehung um die Achse der Befestigungsvorrichtung und der Drehung des Stativs um den Arm 215:180.
In einer vorteilhaften Ausbildung der Erfindung wird vorgeschlagen, dass die Mittel, die bei einer Drehung des Armes um die Achse der Befestigungsvorrichtung das Stativ um die Achse im Fundament drehen, eine Riemen- oder eine Kettentransmission zwischen den Achsen der Befestigungsvorrichtung und des Fundaments sind. Die Mittel können auch Zahnstangen, Paralellogrammarme oder ähnlich mechanische Transmissionen sein.

In einer Weiterentwicklung der Erfindung wird vorgeschlagen, dass das Stativ durch Drehen des Armes in eine dritte Armwinkellage in eine Parklage gebracht werden kann. In einer Parklage ist das Stativ in eine Position gebracht, die ausserhalb des Peripheriebereiches des Untersuchungstisches liegt, was eine Vorbereitung einer Röntgenuntersuchung erleichtert.

Weitere Vorteile und Einzelheiten ergeben sich aus den Unteransprüchen.

Die Erfindung ist nachfolgend anhand eines in den Zeichnungen dargestellten Ausführungsbeispiels näher erläutert. Es zeigen:
- FIG 1: eine Seitenansicht einer Röntgenuntersuchungsausrüstung nach der Erfindung,
- FIG 2: eine Vorderansicht der Röntgenuntersuchungsausrüstung nach der FIG 1 und
- FIG 3 und 4: Draufsichten der Röntgenuntersuchungsausrüstung nach den FIG 1 und 2, bei denen das Stativ in verschiedene Lagen gebracht ist.

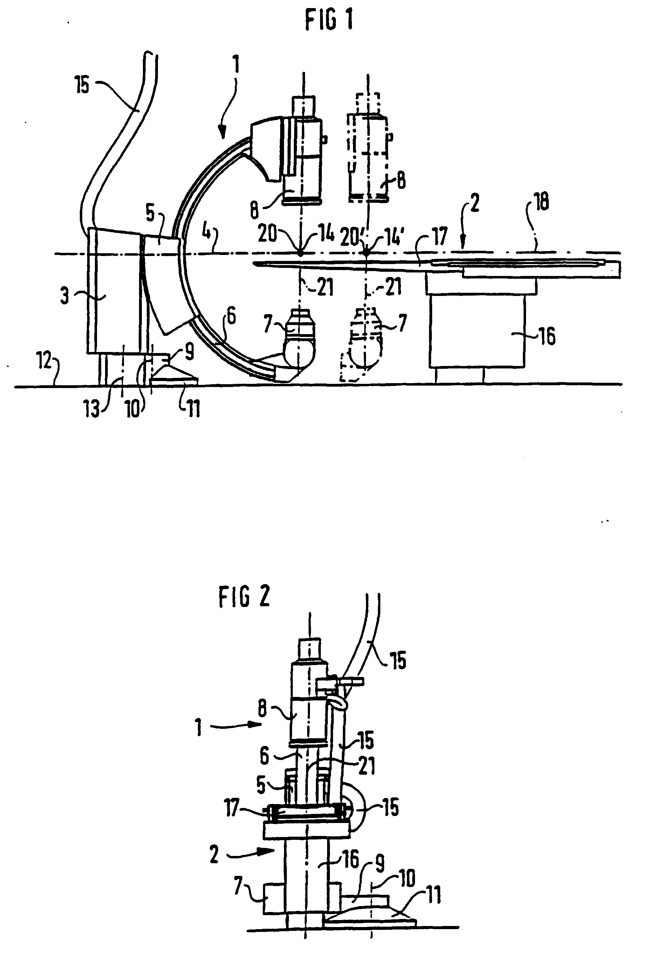

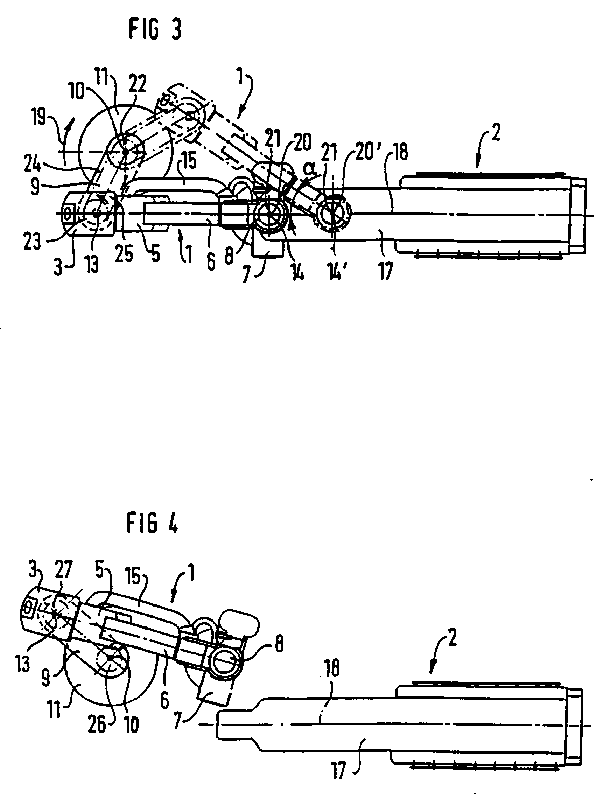

FIG 1 zeigt eine Röntgenuntersuchungsausrüstung mit einem Stativ 1 und einem Untersuchungstisch 2. Das Stativ 1 besteht aus einem Fundament oder einer Säule 3 und einer um eine horizontale Achse 4 drehbaren und mit der Säule 3 verbundenen Halterung 5 für einen C-bogenförmigen Träger 6, an dem eine Röntgenröhre 7 und ein Rezeptor 8 befestigt sind. Die Säule 3 ist über einen Arm 9 um eine Achse 10 einer Befestigungsvorrichtung 11, die mit dem Boden 12 fest verbunden ist, drehbar. Das andere Ende des Armes 9 ist mit einer in der Säule 3 vertikal angeordneten Achse 13 drehbar verbunden. Die Säule 3 ist auch durch bekannte und daher nicht gezeigte Mittel in der Höhe einstellbar. In dieser FIG ist auch ein Zentralkabel 15 für die Stromversorgung des Statives dargestellt. Die FIG zeigt auch, dass die Säule 3 bis auf die niedrigste Lage gefahren ist, bei der der C-Bogen 6 fast am Boden 12 anliegt. Die Röntgenröhre 7 ist gegenüber dem Rezeptor 8 zentriert, was durch die Zentrumlinie 21 dargestellt ist. Wenn das Stativ, wie es in der Fig gezeigt ist, in eine Aufnahmelage gebracht ist, schneidet die Zentrumlinie 21 die fiktive Verlängerung der Achse 4 der Halterung 5 in einem Punkt 14. Dieser Punkt 14 ist ein Isozentrum, um das die Röntgenröhre 7 bzw. der Rezeptor 8, indem der C-Bogen 6 in der Halterung 5 verschoben und um die Achse 4 gedreht werden, geschwenkt werden können. Der Untersuchungstisch 2, der in diesem Ausführungsbeispiel bodenfest ist, besteht aus einem Fuss 16 und einer Tischplatte 17, die zumindest in deren Längsrichtung entlang einer Zentrumlinie 18 verschiebbar ist. Diese Zentrumlinie 18 liegt, wenn das Stativ 1, wie es in diesem Beispiel gezeigt ist, in eine sog.kopfgestellte Lage gebracht ist, achsfluchtend mit der Achse 4 der Halterung 5. Die punktgestrichelte Version der Röntgenröhre 7 bzw. des Rezeptors 8 in dieser FIG, wird in Verbindung mit der FIG 3 näher beschrieben.

In der FIG 2 ist in einer Vorderansicht der Röntgenuntersuchungsausrüstung eine Plazierung des Statives 1 gezeigt, in der dieses in eine kopfgestellte Lage gebracht ist, bei der das Stativ 1 mit dem C-Bogen 6, von oben betrachtet, mit der Zentrumlinie 18 des Tisches 2 achsfluchtend liegt, was in Verbindung mit der FIG 3 näher gezeigt wird. Die FIG zeigt ausserdem, dass die Befestigungsvorrichtung 11 derart plaziert ist, dass deren Achse 10 ausserhalb der Zentrumlinie 18 des Untersuchungstisches 2 liegt.

In der FIG 3, in der die Röntgenuntersuchungsausrüstung in einer Draufsicht dargestellt ist, wird das Stativ 1 in einer in Verbindung mit der FIG 2 bereits beschriebene kopfgestellten Lage gezeigt. Diese FIG zeigt deutlich, dass das Stativ 1 und damit auch die Achse 4 der Halterung 5, von oben betrachtet, mit der Zentrumlinie 18 des Untersuchungstisches 2 achsfluchtend liegt. In dieser FIG ist ausserdem deutlich gezeigt, dass die Befestigungsvorrichtung 11 mit der Achse 10, um die der Arm 9 des Statives 1 gedreht wird, ausserhalb der Zentrumlinie 18 des Untersuchungstisches 2 liegen. Bei einer Drehung des Stativs 1 bzw. des Armes 9 um die Achse 10 in die Richtung des Pfeiles 19, wird das Stativ 1 mit Hilfe von später beschriebenen Mitteln in eine Lage, die einen Winkel α mit der Zentrumlinie 18 des Untersuchungstisches 2 bildet, verschoben. Die punktgestrichelten Konturen des Statives 1 zeigen diese Winkellage. In dieser zuletzt genannten Winkellage schneidet die Zentrumlinie 21 für die Röntgenröhre 7 und den Rezeptor 8 die Zentrumlinie 18 des Untersuchungstisches 2 in einem Punkt 20'. Die Drehung des Statives 1 bzw. der Achse 13 um den Arm 9 im Vergleich zur Drehung des Armes 9 um die Achse 10 der Befestigungsvorrichtung 11 erfolgt mit einer derartigen Übersetzung, dass der Schnittpunkt 20 zwischen der Zentrumlinie 21, der Röntgenröhre 7 und des Rezeptors 8 und der Zentrumlinie 18 des Untersuchungstisches 2 in der zuerst beschriebenen Armwinkellage gegenüber dem Schnittpunkt 20' in der zweiten Armwinkellage, d.h. in der das Stativ einen Winkel mit der Zentrumlinie 18 des Untersuchungstisches 2 bildet, verschoben ist. Die Schnittpunkte 20 bzw. 20' in diesem Beispiel sind in den beiden Armwinkellagen mit den beschriebenen Isozentren 14 bzw. 14' deckungsgleich. Diese Verschiebung des Schnittepunktes 20 und auch die Verschiebung des Isozentrums 14 wird mit Hilfe der punktgestrichelten Konturen der Röntgenröhre 7 und des Rezeptors mit der Zentrumlinie 21 in der FIG. 1 dargestellt. Der Abstand zwischen der Achse 10 der Befestigungsvorrichtung 11 und dem Schnittpunkt 20' in der zweiten Armwinkellage ist durch die beschriebene Stativbewegung um die Achse 10 der Befestigungsvorrichtung 11 grösser als der Abstand zum Schnittpunkt 20 bei der ersten Armwinkellage.

Die Mittel zur Durchführung der beschriebenen Stativbewegung sind z.B. eine Riemen- oder Kettentransmission zwischen der Achse 10 der Befestigungsvorrichtung 11 und der Achse 13 der Säule 3. Der Riemen- oder die Kettentransmission besteht aus einem Zahnrad 22, das mit der Achse 10 fest verbunden ist. Die Achse 10 steht ihrerseits in fester Verbindung mit der Befestigungsvorrichtung 11 und einem Zahnrad 23. Das Zahnrad 23 ist wiederum mit der Säule 3 an der Achse 13 fest verbunden. Zwischen den Zahnrädern 22 und 23 ist ein Riemen oder eine Kette angeordnet. Die Übersetzung zwischen der Drehung des Armes 9 um die Achse 10 der Befestigungsvorrichtung 11 und die Drehung des Statives 1 bzw. der Säule 3 und den Arm 9 ist 215:180. Dieses Übersetzungsverhältnis wird erhalten, indem das Zahnrad 23 auf der Achse 13 der Säule 3 entsprechend grösser als das Zahnrad 22 auf der Achse 10 der Befestigungsvorrichtung 11 ist. Der Arm 9 kann vorzugsweise mit Hilfe eines hier nicht gezeigten Motors, der z.B. an der Befestigungsvorrichtung 11 angeordnet ist, gedreht werden. Wenn der Arm 9 mittels des Motors um die Achse 10 in die Richtung des Pfeiles 19 gedreht wird, wird das Zahnrad 23 dazu gezwungen, auf dem Riemen bzw. auf der Kette 24 zu klettern, so dass das Zahnrad 23 zusammen mit der Säule 3 in die Richtung des Pfeiles 25, was die entgegengesetzte Richtung des Pfeiles 19 ist, gedreht werden. Durch die sukzessive Drehung des Zahnrades 23 bzw. der Säule 3 im Verhältnis zur Bewegung des Armes 9 beschreibt das Stativ 1, wenn es in eine kopfgestellte Lage gebracht ist, zunächst eine vom Untersuchungstisch 2 weggehende und danach eine auf den Untersuchungstisch 2 zugehende, im Vergleich zu dessen Zentrumlinie 18 schräg verlaufende Bewegung, bis das Stativ 1 eine in dieser FIG gezeigte zweite Winkellage einnimmt. Nach einer Röntgenuntersuchung mit dem Stativ 1 in der zweiten Armwinkellage dreht der Motor den Arm 9 in eine im Vergleich zu der beschriebenen Drehbewegung entgegengesetzten Richtung, wobei das Zahnrad 23, wie bereits beschrieben, gezwungen wird, auf dem Riemen oder der Kette 24, aber in entgegengesetzter Richtung zu klettern, so dass das Stativ eine vom Untersuchungstisch 2 weggehende Bewegung beschreibt.

In der FIG 4 ist gezeigt, dass das Stativ 1 eine Parklage einnehmen kann. In dieser Lage des Statives 1 ist ein freier Zugang zum Tisch 2 zwecks Auflegen des Patienten und Zwecks Vorbereitungen vor einer Untersuchung gegeben. Eine solche Lage des Statives 1 wird erreicht, wenn der Arm 9 in einer Position zwischen den bereits beschriebenen ersten und zweiten Armwinkellagen, d.h. zwischen eine kopfgestellte und eine seitlich gestellte Stativlage, gebracht wird.

Wie bereits erwähnt, können statt der beschriebenen Riemen- oder Kettentransmission die Mittel zur Durchführung der Stativbewegungen Zahnstangen, Paralellogrammarme oder ähnliche mechanische Transmissionen sein.

Eine weitere denkbare Lösung zum Steuern des Statives um die Befestigungsvorrichtung 11 ist mit Hilfe von zwei Motoren 26, 27, bei dem der eine Motor 26 auf der Achse 10 der Befestigungsvorrichtung 11 angebracht ist und den Arm 9 um die Achse 10 dreht, und der weitere Motor 27 auf der Achse 13 angebracht ist und die Säule 3 im Verhältnis zum Arm 9 dreht, realisierbar. Die Motoren 26 und 27 sind lediglich in der FIG 4 gezeigt. Durch die Motoren 26, 27 können nun die Bewegungen des Armes 9 bzw. des Statives 1 um die Achse 10 der Befestigungsvorrichtung 11 derart gesteuert werden, dass das Stativ 1 mindestens die bereits beschriebenen beiden Winkellagen und auch eine Parkposition einnehmen kann. Regeleinrichtungen zum Steuern von Motoren in der Weise wie es in diesem Ausführungsbeispiel beschrieben wird, sind in der Robotertechnik bekannt und werden daher in der Anmeldung nicht näher beschrieben.

Durch die Erfindung wird ein Stativ erhalten, dass normalerweise bei einer kopfgestellten Lage einen optimalen Zugang zum Kopf- und Oberkörper des Patienten hat, aber durch die beschriebene asymmetrisch plazierte Rotationsachse 10 in eine Seitenlage gebracht werden kann, die wie beschrieben, auch eine Ganzkörperüberstreichung ohne eine zusätzliche Längsverschiebung des Statives bzw. des Tisches, erlaubt.

## Patentansprüche

1. Röntgenuntersuchungsausrüstung mit einem Stativ (1), bestehend aus mindestens einem Fundament (3), einer um eine horizontale Achse (4) drehbaren und mit dem Fundament (3) verbundenen Halterung (5) für einen gebogenen Träger (6), an dem eine Röntgenröhre (7) und ein Rezeptor (8) befestigt sind, einer Befestigungsvorrichtung (11) für das Stativ (1) und aus einem Arm (9), der das Stativ (1) mit der Befestigungsvorrichtung (11) verbindet, wobei das eine Ende des Armes (9) um eine Achse (10) an der Befestigungsvorrichtung (11) drehbar ist und das andere Ende des Armes (9) mit einer am Fundament (3) angeordneten, vertikalen Achse (13) drehbar verbunden ist, sowie mit einem Untersuchungstisch (17), der zumindest entlang einer Zentrumlinie (18) verschiebbar ist, **dadurch gekennzeichnet**, dass die Befestigungsvorrichtung (11) so plaziert ist, dass deren Achse (10) ausserhalb der Zentrumlinie (18) des Untersuchungstisches (2) liegt und daß Mittel (22, 23, 24) vorgesehen sind, die bei einer Drehung des Armes (9) um die Achse (10) der Befestigungsvorrichtung (11) das Stativ (1) um die Achse (13) am Fundament (3) derart drehen, dass die Zentrumlinie (21) der Röntgenröhre (7) und des Rezeptors (8) die Zentrumlinie (18) des Untersuchungstisches (2) in mindestens zwei Armwinkellagen schneidet, wobei in der ersten Armwinkellage das Stativ (1), von oben betrachtet, mit der Zentrumlinie (18) des Untersuchungstisches (2) achsfluchtend liegt und in der zweiten Armwinkellage das Stativ (1) mit dieser Zentrumlinie (18) einen Winkel (α) bildet.

2. Röntgenuntersuchungsausrüstung nach Anspruch 1, **dadurch gekennzeichnet**, dass die Drehung des Statives (1) um den Arm (9) im Vergleich zur Drehung des Armes (9) um die Befestigungsvorrichtung (11) mit einer derartigen Übersetzung ausgeführt wird, dass der Schnittpunkt (20) zwischen der Zentrumlinie (21) der Röntgenröhre (7) und des Rezeptors (8) und der Zentrumlinie (18) des Untersuchungstisches (2) in der ersten Armwinkellage gegenüber dem Schnittpunkt (20') in der zweiten Armwinkellage verschoben ist.

3. Röntgenuntersuchungsausrüstung nach einem der Ansprüche 1 oder 2, **dadurch gekennzeichnet**, dass der Abstand zwischen der Achse (10) der Befestigungsvorrichtung (11) und dem Schnittpunkt (20') in der zweiten Armwinkellage grösser als der Abstand zum Schnittpunkt (20) in der ersten Armwinkellage ist.

4. Röntgenuntersuchungsausrüstung nach einem der Ansprüche 1 bis 3, **dadurch gekennzeichnet**, dass die Mittel (22, 23, 24) eine Riemen- oder Kettentransmission zwischen den Achsen (10, 13) der Befestigungsvorrichtung (11) und des Fundaments (3) sind.

5. Röntgenuntersuchungsausrüstung nach einem der Ansprüche 1 bis 4, **dadurch gekennzeichnet**, dass die Übersetzung zwischen der Drehung um die Achse (10) der Befestigungsvorrichtung (11) und der Drehung des Stativs (2) um den Arm (9) 215:180 ist.

6. Röntgenuntersuchungsausrüstung nach einem der Ansprüche 1 bis 5, **dadurch gekennzeichnet**, dass das Stativ (1) durch Drehen des Armes (9) in eine dritte Armwinkellage in eine Parklage gebracht werden kann.

7. Röntgenuntersuchungsausrüstung nach einem der Ansprüche 1 bis 6, **dadurch gekennzeichnet**, das die Befestigungsvorrichtung (11) am Boden (12) appliziert ist.

## Claims

1. X-ray examination installation having a stand (1) comprising at least one foundation (3), a holder (5), which can be rotated about a horizontal axis (4) and is connected to the foundation (3), for a curved support (6) on which an X-ray tube (7) and a receptor (8) are fastened, a fastening device (11) for the stand (1), and an arm (9) which connects the stand (1) to the fastening device (11), one end of the arm (9) being rotatable about an axis (10) on the fastening device (11) and the other end of the arm (9) being rotatably connected to a vertical axis (13) arranged on the foundation (3), as well as having an examining table (17) which can be displaced at least along a centre line (18), characterized in that the fastening device (11) is placed such that its axis (10) is situated outside the centre line (18) of the examining table (2) and in that means (22, 23, 24) are provided which upon rotation of the arm (9) about the axis (10) of the fastening device (11) rotate the stand (1) about the axis (13) on the foundation (3) in such a way that the centre line (21) of the X-ray tube (7) and of the receptor (8) intersects the centre line (18) of the examining table (2) in at least two angular arm positions, the stand (1), when viewed from above, being situated in the first angular arm position with its axis flush to the centre line (18) of the examining table (2), and the stand (1) forming in the second angular arm position an angle (α) with this centre line (18).

2. X-ray examination installation according to Claim 1, characterized in that by comparison with the rotation of the arm (9) about the fastening device (11), the rotation of the stand (1) about the arm (9) is carried out with such a transformation ratio that the point of intersection (20) between the centre line (21) of the X-ray tube (7) and of the receptor (8) and the centre line (18) of the examining table (2) in the first angular arm position is displaced by comparison with the point of intersection (20') in the second angular arm position.

3. X-ray examination installation according to one of Claims 1 or 2, characterized in that the spacing between the axis (10) of the fastening device (11) and the point of intersection (20') in the second angular arm position is greater than the spacing from the point of intersection (20) in the first angular arm position.

4. X-ray examination installation according to one of Claims 1 to 3, characterized in that the means (22, 23, 24) are a belt or chain transmission between the axes (10, 13) of the fastening device (11) and the foundation (3).

5. X-ray examination installation according to one of Claims 1 to 4, characterized in that the transformation ratio between the rotation about the axis (10) of the fastening device (11) and the rotation of the stand (2) about the arm (9) is 215:180.

6. X-ray examination installation according to one of Claims 1 to 5, characterized in that the stand (1) can be brought into a parking position by rotating the arm (9) into a third angular arm position.

7. X-ray examination installation according to one of Claims 1 to 6, characterized in that the fastening device (11) is applied to the floor (12).

## Revendications

1. Equipement d'examen radiographique comportant un statif (1), constitué d'au moins un socle (3), d'une fixation (5) tournant par rapport à un axe (4) horizontal, reliée au socle (3) et prévue pour un support (6) incurvé auquel sont fixés un tube (7) à rayons X et un récepteur (8), d'un dispositif (11) de fixation pour le statif (1) et d'un bras (9) qui relie le statif (1) au dispositif (11) de fixation, l'une des extrémités du bras (9) pouvant tourner par rapport à un axe (10) sur le dispositif (11) de fixation et l'autre extrémité du bras (9) étant reliée avec possibilité de rotation à un axe (13) vertical monté sur le socle (3), et comprenant une couchette (17) d'examen qui peut coulisser au moins le long d'une ligne (18) centrale, caractérisé en ce que le dispositif (11) de fixation est placé de telle manière que son axe (10) se trouve à l'extérieur de la ligne (18) centrale de la couchette (2) d'examen et en ce qu'il est prévu des moyens (22, 23, 24) qui, lors d'une rotation du bras (9) par rapport à l'axe (10) du dispositif (11) de fixation, font tourner le statif (1) par rapport à l'axe (13) sur le socle (3) de telle manière que la ligne (21) centrale du tube (7) à rayons X et du récepteur (8) est sécante avec la ligne (18) centrale de la couchette (2) d'examen en au moins deux positions angulaires du bras, le statif (1) étant, vu du haut, en alignement d'axe avec la ligne (18) centrale de la couchette (2) d'examen dans la première position angulaire du bras et le statif (1) faisant un angle (α) avec cette ligne (18) centrale en la deuxième position angulaire du bras.

2. Equipement d'examen radiographique suivant la revendication 1, caractérisé en ce que la rotation du statif (1) par rapport au bras (9) s'effectue, comparé à la rotation du bras (9) par rapport au dispositif (11) de fixation, avec une transmission telle que le point (20) d'intersection entre la ligne (21) centrale du tube (7) à rayons X et du récepteur (8) et la ligne (18) centrale de la couchette (2) d'examen en la première position angulaire du bras est décalée par rapport au point (20') d'intersection en la deuxième position angulaire du bras.

3. Equipement d'examen radiographique suivant l'une des revendications 1 ou 2, caractérisé en ce que la distance entre l'axe (10) du dispositif (11) de fixation et le point (20') d'intersection en la deuxième position angulaire du bras est plus grande que la distance au point (20) d'intersection en la première position angulaire du bras.

4. Equipement d'examen radiographique suivant l'une des revendications 1 à 3, caractérisé en ce que les moyens (22, 23, 24) sont une transmission à courroie ou à chaîne entre les axes (10, 13) du dispositif (11) de fixation et du socle (3).

5. Equipement d'examen radiographique suivant l'une des revendications 1 à 4, caractérisé en ce que la démultiplication entre la rotation par rapport à l'axe (10) du dispositif de fixation et la rotation du statif (1) par rapport au bras (9) est 215:180.

6. Equipement d'examen radiographique suivant l'une des revendications 1 à 5, caractérisé en ce que le statif (1) est mis en une position de stationnement par rotation du bras (9) à une troisième position angulaire de bras.

7. Equipement d'examen radiographique suivant l'une des revendications 1 à 6, caractérisé en ce que le dispositif (11) de fixation est appliqué au sol (12).
